# EUROPEAN PATENT APPLICATION

(11) **EP 4 029 932 A1**
(43) Date of publication of application: **20.07.2022**
(21) Application number: 21151455.9
(22) Date of filing: 13.01.2021
(51) Int. Cl.: C12N 5/073, C12N 5/071

(54) **BLASTOCYST-LIKE CELL AGGREGATE AND METHODS**

(71) Applicant: IMBA-Institut für Molekulare Biotechnologie GmbH, 1030 Wien (AT)
(72) Inventor: RIVRON, Nicolas, Wien 1150 (AT); KAGAWA, Harunobu, Wien 1030 (AT); JAVALI, Alok, Wien 1040 (AT); HEIDARI KHOEI, Heidar, Wien 1030 (AT)
(74) Representative: SONN Patentanwälte OG

(57) **Abstract**

The present invention provides a method of generating a blastoid or a blastocyst-like structures by aggregation and culture of cells comprising culturing an aggregate of human pluripotent stem cells (hPSCs) and trophoblast cells in a medium comprising a HIPPO pathway inhibitor in a 3D culture; blastoids and a blastocyst-like cell aggregates obtainable by said methods and uses thereof; as well as similar treatments of blastocyst for in vitro fertilization that increase blastocyst development and implantation; as well as similar contraceptive treatment of human embryos that decrease blastocyst development and implantation.

## Description

The present invention relates to the generation of blastocyst-like structures by aggregation and culture of cells.

### Background of the invention

Understanding of human early embryonic development and implantation are extremely limited due to the small number of studied human embryos and to the difficulties to experimentally manipulate them physically and genetically. Other experimental model organisms have been used for studying these early development and implantation mechanisms, typically mice. However, it has now become clear that there are important differences between humans and other species in terms of morphology and molecules at play, which renders the study of human embryogenesis necessary. Altogether, there is a compelling need for in vitro alternatives to the use of human embryos for research. Models are needed that can be made widely available, are amenable to easy genetic manipulations and high-throughput drug screens.

Accordingly, it is a goal of the invention to provide such models.

### Summary of the invention

The present invention provides a method of generating a blastoid or a blastocyst-like cell aggregate comprising culturing an aggregate of human pluripotent stem cells (hPSCs) and trophoblast cells in a medium comprising a HIPPO pathway inhibitor in a 3D culture. These blastoids can be used for high-throughput genetic or drug screens in the context of drug development. This method can also be used for triggering a pregnancy. The components of the medium and molecules revealed by the use of blastoids can also be used to modulate the behaviour of blastocysts, for example during in vitro fertilization procedure, in order to improves blastocyst development and implantation.

The invention further provides a kit suitable for culturing a blastoid, comprising a HIPPO pathway inhibitor, a MEK inhibitor, and a TGF-beta inhibitor. One or more of these compounds can be combined in a medium for culturing human pluripotent stem cells (hPSCs).

The invention further provides blastoids and a blastocyst-like cell aggregates obtainable by said methods.

Also provided is a blastoid or blastocyst-like cell aggregate comprising an outer epithelial monolayer of trophoblast-like cells, preferably characterized by the expression of for example GATA3 and CDX2, surrounding at least one fluid-filled cavity and at least one inner cluster of cells comprising epiblast-like, preferably characterized by the expression of for example Nanog and Oct4, and hypoblast-like cells, preferably characterized by the expression of for example GATA4, wherein the outer epithelial monolayer comprises polar-like trophoblasts that express NR2F2.

The invention further provides an in vitro method of increasing or testing the potential of a blastoid or a blastocyst to implant into a layer of endometrial cells, comprising stimulating the endometrial cells with a Wnt inhibitor, preferably XAV939 and/or LF3, contacting the blastoid or blastocyst with the layer of stimulated endometrial cells; in the method of testing further measuring the level of attachment, invasion, and differentiation to the trophoblasts, blastoid or blastocyst into the endometrium cells.

The invention further provides a Wnt inhibitor for use in a method of increasing the chance of a blastocyst implantation, for example during an in vitro fertilization procedure, comprising contacting the blastocyst with an endometrium in the presence of the Wnt inhibitor or stimulating the endometrium with a Wnt inhibitor before transferring the blastocyst in utero or to the endometrium.

Related thereto, the invention provides a method of increasing the chance of a blastocyst to implant, for example during an in vitro fertilization procedure, comprising contacting the blastocyst with an endometrium in the presence of the Wnt inhibitor or stimulating the endometrium with a Wnt inhibitor before transferring the blastocyst in utero or to the endometrium. Also provided is the use of a Wnt inhibitor for manufacturing a pharmaceutical composition for mediating blastocyst implantation, e.g. during an in vitro fertilization procedure, that comprises contacting the blastocyst with an endometrium in the presence of the Wnt inhibitor or stimulating the endometrium with a Wnt inhibitor before transferring the blastocyst in utero or to the endometrium.

Further provided is a HIPPO pathway inhibitor for use in a method of producing a blastocyst suitable for implantation, for example to improve the quality of a blastocyst during an in vitro fertilization, comprising treating an embryo in an early stage, selected from the morula stage or blastocyst stage until a mature blastocyst stage, with the HIPPO pathway inhibitor and letting the embryo in a morula stage grow into the blastocyst stage or letting the embryo in the blastocyst stage grow into a more mature blastocyst stage.

Related thereto, the invention provides a method of producing a blastocyst suitable for in vitro fertilization, comprising treating an embryo in an early stage, selected from 1-cell stage, 2-cells stage, 4-cells stage, 8-cells stage or 16-cells stage, the morula stage or blastocyst stage until a mature blastocyst stage, with the HIPPO pathway inhibitor and letting the embryo in a morula stage grow into the blastocyst stage or letting the embryo in the blastocyst stage grow into a more mature blastocyst stage. The 1-cell stage, 2-cells stage, 4-cells stage, 8-cells stage or 16-cells stage can also be referred to as the cleavage stages. Also provided is the use of a HIPPO pathway inhibitor for the manufacture of a pharmaceutical composition for producing a blastocyst suitable for implantation, for example to improve the developmental potential of a blastocyst during an in vitro fertilization procedure, which comprises treating an embryo in an early stage, selected from the 1-cell stage, 2-cells stage, 4-cells stage, 8-cells stage or 16-cells stage, morula stage or blastocyst stage until a mature blastocyst stage, with the HIPPO pathway inhibitor and letting the embryo in a 1-cell stage, 2-cells stage, 4-cells stage, 8-cells stage or 16-cells stage, or morula stage grow into the blastocyst stage or letting the embryo in the blastocyst stage grow into a more mature blastocyst stage.

All aspects of the invention are related and any disclosure of specific embodiments for one aspect also relate to other aspects. E.g. a disclosure of treatments of a blastoid or a blastocyst-like cell aggregate in vitro can also be done for the treatments in vivo for preparation of blastocyst for preparation in an in vitro fertilization procedure and the preceding treatments steps. Any compound described for the inventive methods may be part of the kit. The components of the kit and the kit may be used in the inventive methods and treatments.

### Detailed description of the invention

The invention includes a method to form blastocyst-like cell aggregates, generally termed blastoids, from human pluripotent stem cells (hPSCs). Contrary to in vivo blastocysts, blastoids can be produced in large numbers and are amenable to genetic and drug screens, while alleviating some ethical concerns related to the manipulation of human embryos given that the artificial blastocyst-like cell aggregates and blastoids are not able to form or develop into human embryos.

Blastocyst-like cell aggregates and blastoids are human embryo models that have an important potential for biomedical discoveries, including for drug safety/efficacy and for therapies of early pregnancy (e.g., improving in vitro fertilization procedures and contraceptives).

As used herein, the terms blastocyst-like cell aggregates and blastoids are used interchangeably to reflect the tissues obtainable by the inventive methods that model blastocyst without being true blastocyst. The term blastocyst is reserved to such embryos.

Blastoids recapitulate the three-dimensional morphological and molecular signatures of the human blastocyst including the concomitant specification and spatial organization of tissues reflecting the three founding lineages that form the whole organism, namely the trophectoderm, the epiblast and the hypoblast.

The invention also describes treating (human) blastocysts to prepare them for improved implantation chances, e.g. during in vitro fertilization (IVF) procedures or treating a patient after a natural conception to improve the chances of pregnancy. Such treatments may be medical or therapeutic in nature to treat the human embryo or the recipient mother. For such methods, the invention also relates to manufacturing pharmaceutical compositions with the compounds for the treatment (e.g. a HIPPO pathway inhibitor) or said compounds for use in the treatment. Human embryos as such or their uses for industrial or commercial purposes may not be part of the invention.

As a core aspect, the present invention provides a method of generating a blastoid or a blastocyst-like cell aggregate comprising culturing an aggregate of human pluripotent stem cells (hPSCs) and trophoblast cells in a medium comprising a HIPPO pathway inhibitor in a 3D culture. Preferably, the human pluripotent stem cells (hPSCs) are surrounded or get surrounded by the trophoblast cells.

According to the invention, the formation of blastoids according to the invention achieves forming three-dimensional aggregates of hPSCs and modulating the activity of the HIPPO pathway, which triggers the concomitant specification and three-dimensional self-organization of epiblast-, trophectoderm- and hypoblast-like cells, along with the formation of an embryonic-abembryonic axis. Accordingly, a HIPPO pathway inhibitor is used as a core aspect of the invention to generate the blastoids.

The invention provides for the first time the induction of the concomitant formation of the three cell types of (i) epiblast-like, (ii) trophectoderm-like, and (iii) hypoblast-like cells from hPSCs and their self-organization into structures morphologically and molecularly similar to the human blastocyst, thereby recapitulating the 3D morphological change constrained by the concomitant cell lineage segregation, morphogenesis, and maturation of tissues reflecting the trophectoderm, epiblast and hypoblast.

The resulting blastoids can actively interact with a layer of endometrial cells in vitro or the endometrium, the lining cells of the uterus in vivo, when it is made receptive, for example upon stimulation with Estrogen, Progesterone, cAMP, and the Wnt-inhibitor XAV939 and/or LF3. Similar to blastocysts, the attachment, invasion, and differentiation of blastoids to the endometrial cells occurs predominantly via the polar trophectoderm, meaning the trophoblasts that juxtapose the epiblast cells. Upon implantation, the polar trophoblasts of the blastoids proliferate, differentiate, and produce human chorionic gonadotropin, the hormone used to signify a clinical pregnancy.

Accordingly, the inventive method is useful for
1. genetic and drug screens for understanding and managing early pregnancy;
2. development of contraceptive and fertility drugs;
3. development of IVF culture conditions;
4. in vitro toxicity/safety assays for drug development;
5. in vitro formation of specific cells, tissues, and organs for in vitro assays and in vivo transplantations.

The present invention comprises the step of culturing an aggregate of human pluripotent stem cells (hPSCs) and of trophoblast cells in a medium comprising a HIPPO pathway inhibitor in a 3D culture. Preferably, the human pluripotent stem cells (hPSCs) get surrounded by the trophoblast cells in this method step or human pluripotent stem cells (hPSCs) are surrounded by the trophoblast cells through a preceding step, e.g. an optional step of culturing aggregated hPSCs in a medium comprising a HIPPO pathway inhibitor, in which trophoblast cells may form surrounding the hPSCs.

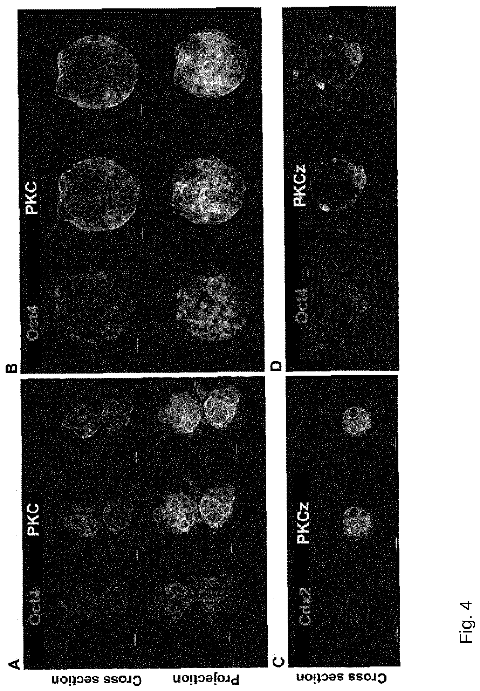

The term "HIPPO pathway inhibitor" and "HIPPO pathway antagonist" are used interchangeably herein. This term refers to a compound that reduces activity of the HIPPO pathway. The HIPPO pathway is reviewed in Gumbiner and Kim, Journal of Cell Science (2014) 127, 709-717 (incorporated herein by reference). The HIPPO pathway exercises inhibitory action on the ability of Hippo-Yes-associated protein (YAP) to shuttle to the nucleus. One such inhibitory action is through phosphorylation of YAP, which prevents YAP from entering the nucleus. Compounds that prevent or reduce YAP phosphorylation in a cell are thus suitable HIPPO pathway inhibitors. By inhibiting the HIPPO pathway, the inhibition on YAP is removed or reduced, leading to an increased activity of YAP in the nucleus and by consequence usually to cell proliferation (see e.g. Fig. 4 of Gumbiner and Kim). As such the HIPPO pathway inhibitor of the invention may also be a YAP activator, i.e. leading to increased YAP activity in the nucleus. As such, a HIPPO pathway inhibition includes YAP activation and HIPPO pathway inhibitor include YAP activators. An example YAP activation is e.g. overexpression of YAP in a cell, such as by using a recombinant nucleic acid expressing YAP as YAP activator. Such a nucleic acid, can be administered to a cell, e.g. using a vector, for YAP activation. A Hippo pathway inhibitor is XMU-MP-1 (Triastuti et al., Br J Pharmacol. 2019; 176: 3956-3971), which is preferably used in lower concentrations according to the inventive methods of preparing a blastoid and preparing a blastoid or blastocyst for implantation into an endometrium. XMU-MP-1 is an exceptionally potent Hippo pathway inhibitor. High amounts of XMU-MP-1, e.g. more than 1 µM or about 2 µM and more in culture, may be used to induce the formation of trophectoderm-like cells and to form a blastoid with a limited number of or no inner cells (so-called trophosphere). XMU-MP-1 is a strong inhibitor of the Hippo pathway with stronger and more long-lasting effects than LPA. As a result, the aggregate largely forms trophoblasts at the expense of forming epiblasts and hypoblast cells. Blastoids with inner cells form at lower amounts of XMU-MP-1, e.g. about 1 µM or less in culture. With optimization of concentration of a Hippo pathway inhibitor, the length of exposure of the cells to the Hippo inhibitor, the combination of the Hippo inhibitor with additional molecules, and the initial number of cells the result can be controlled in general. XMU-MP-1 is an inhibitor of MST1/2. The invention provides the use of XMU-MP-1 or an inhibitor of MST1/2 in a method of generating a trophosphere (which may lack inner cells) that does not have the potential to attach and invade endometrial cells - this method may be in vitro; and/or in a method of contraception. Sufficient amount of XMU-MP-1 and time of exposure of the cells to XMU-MP-1 may be used to obtain a trophosphere, e.g. 2 µM or more and an exposure of 4 days or more. Also provided is a MST1/2 inhibitor, preferably XMU-MP-1 for use as a contraceptive.

A preferred HIPPO pathway inhibitor is a ligand of the lysophosphatidic acid receptor (LPAR), in particular preferred lysophosphatidic acid itself (LPA). A further preferred HIPPO pathway inhibitor and a ligand of the lysophosphatidic acid receptor is NAEPA or OEA-P (oleoyl ethanolamide phosphate), N-[2-(phosphonooxy)ethyl]-9Z-octadecenamide. These are a lysophosphatidic acid (LPA) mimetics. The ligand of the LPAR may be an activator or agonist of the LPAR. As further or alternative LPAR ligands any derivative of LPA may be used. Derivatives of LPA are preferably compounds of formula 1: 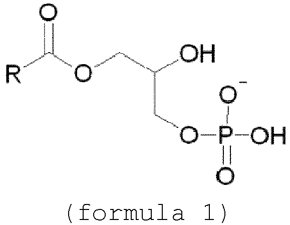 wherein R is a C₈-C₂₄-alkyl, a C₈-C₂₄-alkenyl or C₈-C₂₄-alkynyl. Preferably R is a C₉-, C₁₀-, C₁₁-, C₁₂-, C₁₃-, C₁₄-, C₁₅-, C₁₆-, C₁₇-, C₁₈-, C₁₉-, C₂₀-, C₂₁-, C₂₂-, C₂₃-alkenyl, -alkyl or -alkynyl. A preferred compound is (2-hydroxy-3-phosphonooxypropyl) (Z)-octadec-9-enoate.

The LPAR is preferably LPAR1, LPAR2, LPAR3, LPAR4, LPAR5 or LPAR6. In particular preferred the LPAR is LPAR2.

Further LPAR ligands are GRI977143 and any derivatives thereof as disclosed in WO 2014/036038 A1 (incorporated herein by reference). Such ligands include compounds of formula 2: 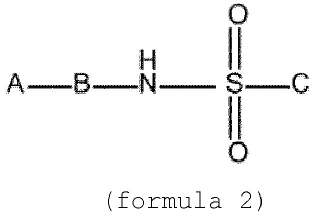
wherein A is 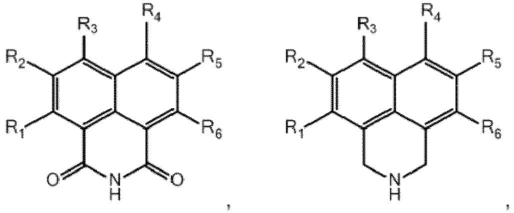 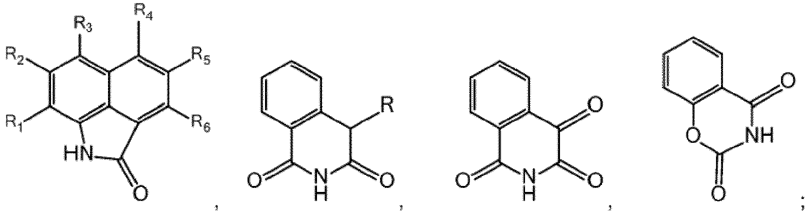
R is H or substituted or unsubstituted phenyl;
R₁, R₂, R₃, R₄, R₅, and R₆ are independently H, NO₂, Br, Cl, or OCH₃;
B is C₂-C₈-alkyl or -alkenyl; and C is 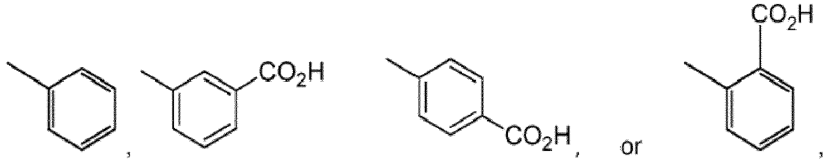 optionally substituted with F, CI, Br, NO₂, NH₂, OCH₃, CH₃, CO₂H, or phenyl. For example, the compound can be 2-((9-oxo-9*H*-fluoren-2-yl)carbamoyl)benzoic acid, 2-((3-(1,3-dioxo-1*H-*benzo[de]isoquinolin-2(3*H*)-yl)propyl)thio)benzoic acid, 4,5-dichloro-2-((9-oxo-9*H*-fluoren-2-yl)carbamoyl)benzoic acid or 2-((9,10-dioxo-9,10-dihydroanthracen-2-yl)carbamoyl)benzoic acid.

Alternative or combinable HIPPO pathway inhibitors may be a Mst1 inhibitor, a Mst2 inhibitor or a combined Mst1 and Mst2 inhibitor, such as XMU-MP-1 (Triastuti et al., *supra*) or a Lats kinase inhibitor, such as TRULI (Kastan et al., bioRxiv, 2020, doi.org/10.1101/2020.02.11.944157). The Lats kinase inhibitor may be an ATP-competitive inhibitor of Lats kinases. Lats kinase is involved in YAP phosphorylation (Gumbiner et al, *supra*) and inhibition of Lats activity therefore decrease YAP inactivation by phosphorylation and increases YAP activity in the nucleus.

The HIPPO pathway inhibitor may be a YAP activator, in particular a YAP activator that reduces or prevents YAP phosphorylation and/or facilitates entry of YAP into the nucleus of a cell.

A 3D culture is a culture that allows tissue development in all three dimensions. Contrary thereto, in a 2D culture cells are induced to grow attached onto a surface and are deterred from growing away from said surface, with such growth not necessarily being excluded. 2D culturing may induce cell layer formation, such as mono-layers, bilayers or multilayers and/or two-dimensional cell expansion; 3D culturing usually allows growth in all directions equally, wherein of course the cell tissues are allowed to develop a tissue directionality or axis by themselves. Layer formation in 2D cultures may be induced by gravity and/or adhesion between cells or to a surface. Conditions for 2D and 3D cultures may be influenced by the type of surface, e.g. adherent surface for 2D cultures and non-adherent surface for 3D cultures, the medium, the lack (2D) or presence (3D) or a scaffolding 3D matrix, such as a gel structure, e.g. hydrogel. 2D culture may comprise feeder cells as adherent surface.

A medium for growing the cells and tissue allows unhindered development to a blastoid. A medium may comprise nutrients, such as one or more carbohydrates, amino acids and salts. An example medium is B27N2 medium (Sünwoldt et al., Front. Mol. Neurosci. 10, 2017:305). The medium preferably comprises insulin. Alternatively or in combination, the medium may comprise holotransferin, selenite, corticosterone or progesterone, retinol or a combination thereof. Such a medium may also be provided with the inventive kit.

The inventive method comprises providing an aggregate of human pluripotent stem cells (hPSCs) and trophoblast cells. hPSCs are pluripotent cells that are able to differentiate into the trophectoderm-like tissue, epiblast-like tissue and hypoblast-like tissue. The suffix "-like" indicates that although the tissues will resemble trophectoderm, epiblast and hypoblast, respectively, these tissues will usually not develop identically as in an in vivo situation since the inventive blastoids are still artificial constructs. However, the "-like" tissues of the blastoid of the invention will usually express similar expression markers as the in vivo counterparts and can be identified similarly.

The aggregate of human pluripotent stem cells (hPSCs) and trophoblast cells can be provided by common techniques, such as disclosed in WO 2014/171824 A1 or in Okae et al., Cell Stem Cell 22, 2018: 50-63 (both incorporated herein by reference). In a preferred method according to the invention, the aggregate of hPSCs and trophoblasts can be generated by culturing aggregated hPSCs in a medium comprising any one selected from a HIPPO pathway inhibitor, a MEK inhibitor and a TGF-beta inhibitor. Preferably a MEK inhibitor and a TGF-beta inhibitor are used. Preferably also a HIPPO pathway inhibitor, is used.

MEK inhibition, by e.g. a MEK inhibitor, can be used for inducing the formation of trophoblasts from hPSCs as disclosed in Guo et al., bioRxiv 2020, doi.org/10.1101/2020.02.04.933812. If cell aggregates with trophoblast cells are already provided, a MEK inhibition is not needed. If no trophoblast cells are present, these can be formed by using the MEK inhibitor.

The MEK inhibitor is preferably PD0325901. PD0325901 can be a compound of formula 3: 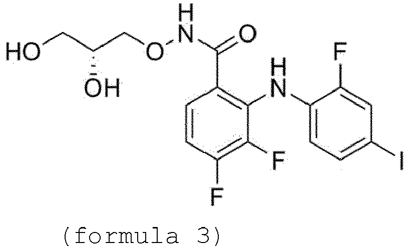 Further or alternative MEK inhibitors may be selected from Selumetinib, Mirdametinib, Trametinib, U0126-EtOH, PD184352 (CI-1040), PD98059, Pimasertib, TAK-733, AZD8330 (ARRY704), Binimetinib, PD318088, SL327, Refametinib, GDC-0623 (G-868), Cobimetinib.

The TGF-beta inhibitor may be an inhibitor of TGF-β type I receptor. The TGF-beta inhibitor is preferably A83-1 (A83-01, A-83-01). A83-1 can be a compound of formula 4: 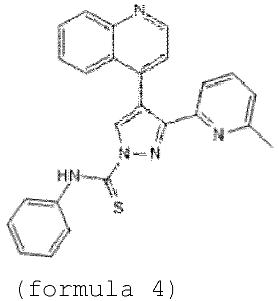 Further or alternative TGF-beta inhibitors may be selected from SD-208, GW788388, SRI-011381, TP0427736, RepSox (E-616452, SJN 2511), LY2109761, SB505124, BIBF-0775, LY 3200882, Galunisertib (LY2157299), Vactosertib (TEW-7197, EW-7197), LY364947 (HTS 466284), SB525334, ITD-1.

The aggregated hPSCs (for the above step of generating an aggregate of hPSCs and trophoblast cells) can be formed by seeding hPSCs and aggregating the seeded hPSCs by culturing in a growth medium. The hPSCs to be seeded are preferably dissociated hPSCs. hPSCs may e.g. be dissociated by trypsinization. Dissociated hPSCs are not aggregated into one combined tissue. However, they are able to aggregate later during development in the inventive method.

These method steps to generate different stages and intermediaries during the formation of the inventive blastoid can be combined, e.g. when it is desired to produce the aggregate of hPSCs and trophoblast cells in situ. As such, the blastoid can be formed from hPSCs in one culture. The same basic media may be used for growth and nutrient supply (e.g. B27N2 or others) but different additional compounds are used during different steps. The inventive method may comprise the combination of
(i) seeding hPSCs and aggregating the seeded hPSCs by culturing in a growth medium to form the aggregated hPSCs,
(ii) culturing aggregated hPSCs in a medium comprising a MEK inhibitor and a TGF-beta inhibitor to generate the hPSCs and trophoblast cells; optionally a HIPPO pathway inhibitor is also used in step (ii);
(iii) culturing the aggregate of hPSCs and trophoblast cells in a medium comprising a HIPPO pathway inhibitor in a 3D culture to generate the blastoid or a blastocyst-like cell aggregate.

The invention includes combinations of steps (i), (ii) and (iii), but also a combination of steps (ii) and (iii), the latter e.g. if aggregated hPSCs can be provided (without the surrounding trophoblast cells that are the starting point for step (iii)) .

In step (iii), the MEK inhibitor and/or the TGF-beta inhibitor may not be used. In step (i) or in step (ii) the HIPPO pathway inhibitor may not be used. Also in step (i), alternatively or in combination, the MEK inhibitor and/or the TGF-beta inhibitor may not be used. In step (ii), the use of the TGF-beta inhibitor and MEK/ERK inhibitor is sufficient to form blastoids in step (iii).

The aggregated hPSCs (for the step of generating an aggregate of hPSCs and trophoblast cells) are preferably formed by seeding hPSCs and aggregating the seeded hPSCs by culturing in a growth medium with culturing in a growth medium for 12 to 64 hours as a preferment of step (i). In a preferred embodiment of step (i), combinable or as alternative, the growth medium comprises a ROCK inhibitor. A preferred ROCK inhibitor is Y27632 (Y-27632). Further or alternative ROCK inhibitors may be selected from ZINC00881524, Thiazovivin, Fasudil(HA-1077), GSK429286A (RHO-15), RKI-1447, Azaindole 1 (TC-S 7001), GSK269962A HCl (GSK269962B, GSK269962), Hydroxyfasudil (HA-1100), Netarsudil (AR-13324), Ripasudil (K-115), Y-39983 (Y-33075), KD025 (SLx-2119). The ROCK inhibitor increases or improves aggregation of the seeded hPSCs.

In a preferment, culturing of seeded hPSCs in the growth medium (e.g. step (i) mentioned above) comprises seeding 1 to 200 hPSCs, preferably 20 to 150 hPSCs, in particular preferred 30 to 60 hPSCs, in a vessel and growing said seeded hPSCs in the growth medium. This number of cells leads to an optimal blastoid formation in later steps.

In preferred embodiments, the treatment and or growth of the seeded hPSCs is or has been done in a 2D culture environment. 2D cultures may lead to a two-dimensional cell expansion as mentioned above.

Preferably, the hPSCs during the 2D culture, (before the 3D culture), have been treated with a MEK inhibitor and/or a PKC inhibitor, e.g. as described in Guo et al., Development 2017, doi: 10.1242/dev.146811. A Wnt inhibitor and a STAT activator may also be used in combination or alternatively as mentioned above.

In preferred embodiments, the PKC inhibitor is selected from G66983 (GOE 6983) and Ro-31-8425 or a combination thereof. Further or alternative PKC inhibitors may be selected from Enzastaurin (LY317615), Sotrastaurin (AEB071), Mitoxantrone (NSC-301739), Staurosporine (CGP 41251), Bisindolylmaleimide I (GF109203X, GO 6850), Bisindolylmaleimide IX (Ro 31-8220), LXS-196 (IDE-196), VTX-27, Midostaurin (pkc412, CGP 41251), Chelerythrine, Go6976 (PD406976).

In preferred embodiments, the 2D cultured hPSCs further is or have been treated with a Wnt inhibitor and/or a STAT agonist. Such a treatment results in more naive hPSCs or hPSCs in a ground state. Such naive or ground state hSPCs are preferably used as hPSCs in the inventive method in step (i). Such a treatment to produce more naive or ground state hPSCs is e.g. disclosed in Takashima et al., Cell 158(6), 2014: 1254-1269 or WO 2016/027099 A2 (both incorporated herein by reference). The STAT agonist is preferably a STAT3 agonist, e.g. LIF.

An example Wnt inhibitor is XAV939. XAV939 may be a compound of formula (5): 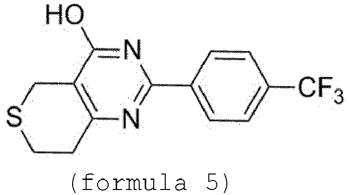 Further or alternative Wnt inhibitors may be selected from LF3, PKF118-310, Wnt3A, Adavivint (SM04690), CCT251545, PNU-75654, IWP-2, IWP-3, IWR-1-endo, iCRT3, WIKI4, ICG-001, XAV-939 (NVP-XAV939), LGK-974 (NVP-LGK974, WNT974), MSAB, KYA1797K, JW55, or combinations thereof. LF3 and/or XAV939 are particular preferred. Particular preferred Wnt inhibitors for all embodiments of the invention are XAV939, IWP2, PNU74654 and LF3.

In a particular preferred embodiment, the hPSCs may be pretreated in a medium containing inhibitors of MEK, Wnt, PKC, and an against or activator of STAT (e.g. LIF) as e.g. described in Guo et al., Development 2017, doi: 10.1242/dev.146811 and Takashima et al., *supra.* A medium termed PXGL maintains hPSCs in a more naive state. This more naive state improves the formation of blastoids. Many culture conditions to make hPSCs naive are known in the art and can be used according to the invention, e.g. as described in WO 2016/027099 A2.

In preferred embodiments, the aggregated cells (e.g. step (ii) mentioned above) are cultured for at least 1 day, preferably at least 2 days.

The above embodiments are preferably combined, e.g. in an example, a culture starting from hPSCs comprises culturing the cells at least 5 days: the first day is in medium without small molecule inhibitors (without a MEK inhibitor (e.g. PD0325901), a TGF-beta inhibitor (e.g. A83-01), an activator of STAT (e.g. LIF), and a HIPPO pathway inhibitor (e.g. LPA)) as discussed above (except for a ROCK inhibitor, e.g. Y27632, which favors the aggregation of the cells). This is an example of step (i) and anything said above for step (i) also applies here. On the second day, the treatment with a MEK inhibitor (e.g. PD0325901), a TGF-beta inhibitor (e.g. A83-01), an activator of STAT (e.g. LIF), a ROCK pathway inhibitor, and a HIPPO pathway inhibitor (e.g. LPA) is started. This is an example of step (ii) and anything said above for step (ii) also applies here. The same medium is used on the third day (step (ii)). On the fourth day, the cells/aggregates are cultured in a medium containing only the ROCK pathway inhibitor and the HIPPO pathway inhibitor (e.g. LPA) from the discussed small molecule inhibitors, e.g. the MEK inhibitor (e.g. PD0325901), a TGF-beta inhibitor (e.g. A83-01), an activator of STAT (e.g. LIF) are not used anymore. This is an example of step (iii) and anything said above for step (iii) also applies here. The blastoids are usually fully formed on day 5.

By modifying growth conditions, preferred times for each step may vary. In preferred embodiments, step (i) is for 18 to 48 hours; preferably step (ii) is for 36 to 92 hours; preferably step (iii) is for 18 to 48 hours. These times are also preferred embodiments if step (i) or steps (i) and (ii) are not done, e.g. if aggregated hPSCs or an aggregate of hPSCs and trophoblast cells are used as a starting point.

The hPSCs (human pluripotent stem cells) are preferably not human totipotent cells, i.e. they are not human embryos.

The hPSCs may be from any cell line. Preferably the cell line is selected from hESC H9, Shef6, HNES1, hiPSC cR-NCRM2, and hiPSC niPSC16.2.b.

Preferably, the cells, aggregated hPSCS or the aggregate of hPSCs and trophoblast cells, selected independently, are seeded or placed into a microwell. A microwell may be used to control the number of cells. The microwell may be in an array to allow a plurality of parallel blastoid formations. Preferably, at least 2, e.g. 3, 4, 5, 6, 7, 8, 9 or 10 or more, such as 50 or more blastoids are created in parallel by the inventive method.

Preferably, the 3D culture, e.g. the culturing of the aggregate of hPSCs and trophoblast cells, is done by culturing in a non-adherent vessel, preferably by culturing in microwells, especially preferred by microwells comprising a non-adherent surface made of hydrogel.

In preferred embodiments, the medium for culturing in a 3D culture, in particular the culturing the aggregate of hPSCs and trophoblast cells (such as step (iii) mentioned above), and/or the medium for culturing aggregated hPSCs (such as step (ii) mentioned above) further comprises a STAT3 agonist. The STAT3 agonist is preferably leukemia inhibitory factor (LIF). LIF is preferably human LIF.

In preferred embodiments the hPSCs and trophoblasts (e.g. step (iii) mentioned above) are cultured for at least 16 hours, preferably at least 20 hours, even more preferred at least 1 day, possible are also at least 2 days.

Preferably, the culturing the cells, in particular the hPSCs and trophoblast cells, is at least until formation of a trophectoderm-like tissue, an epiblast-like tissue and a hypoblast-like tissue out of the aggregate of hPSCs and trophoblasts. Alternatively or in combination, culturing may be at least until formation of an embryonic-abembryonic axis.

Alternatively, the culturing of the cells, in particular the hPSCs and trophoblast cells, is done in the presence of the MST1/2 inhibitor XMU-MP-1 that favours the formation of trophoblasts and disfavours the formation or the maintenance of the epiblast-like cells and hypoblast-like cells, when in sufficient concentration of XMU-MP-1, until the formation of trophoblast cysts that contain a smaller inner cluster or do not contain an inner cluster of epiblast-like and hypoblast-like cells. These trophoblast cysts are termed XMU-MP-1-Trophospheres.

The invention provides the use of XMU-MP-1 or an inhibitor of MST1/2 in a method of generating a trophosphere (which may lack inner cells) - this method may be in vitro; and/or in a method of contraception. The MST1/2 inhibitor, preferably XMU-MP-1, may be used for contraception. Also provided is a MST1/2 inhibitor, preferably XMU-MP-1 for use as a contraceptive. The MST1/2 inhibitor, preferably XMU-MP-1 may be administered to a patient 1 to 9 days after conception, preferably 2 to 7 days after conception, in particular preferred 3 to 6 days after conception.

Alternatively, the culturing the cells, in particular the hPSCs and trophoblast cells, is done in the presence of the STAT inhibitor SC144 that disfavours the formation and maintenance of the epiblast-like cells and hypoblast-like cells until the formation of trophoblast cysts that do not contain an inner cluster of epiblast-like cells and hypoblast-like cells or contain a decreased number of epiblast-like cells and hypoblast-like cells. These trophoblast cysts are termed SC144-Trophospheres. As described above, a STAT agonist may be used in certain steps of the inventive method. In order to prevent or limit the formation of a blastoid with inner cells, a STAT inhibitor, like SC144, can be used. The STAT inhibitor, preferably SC144, may be used for contraception. Also provided is a STAT inhibitor, preferably SC144, for use as a contraceptive. The STAT inhibitor, preferably SC144, may be administered to a patient 1 to 9 days after conception, preferably 2 to 7 days after conception, in particular preferred 3 to 6 days after conception.

Trophoblasts are cells that form the outer layer of a blastocyst or blastoid, and would develop into a large part of the placenta in vivo. The term trophectoderm describes the epithelial cystic tissue that forms the outer layer of the blastocyst. In the blastoid, an outer tissue resembles such trophectoderm and is referred to as trophectoderm-like tissue.

Epiblast is one of two distinct layers arising from the inner cell mass in the mammalian blastocyst. It derives the embryo proper through its differentiation into the three primary germ layers, ectoderm, mesoderm and endoderm, during gastrulation. In the blastoid, an inner tissue develops that resembles such epiblast and is referred to as epiblast-like tissue.

The hypoblast, is one of two distinct layers arising from the inner cell mass in the mammalian blastocyst. The hypoblast gives rise to the yolk sac, which in turn gives rise to the chorion. In the blastoid, an inner tissue develops that resembles such hypoblast and is referred to as hypoblast-like tissue.

The formation of the embryonic-abembryonic axis is a preferred embodiment as the blastocyst and blastoids implants via the trophoblasts that juxtapose the inner aggregate of epiblasts/hypoblasts. These so-called polar trophoblasts are characterized by the expression of NR2F2+. Preferably, the inventive blastoid comprises polar trophoblasts expressing NR2F2+.

Preferably the cells, in particular the hPSCs and trophoblast cells, are cultured at least until formation of a three-dimensional cell aggregate with an overall diameter of at least 100 µm, preferably at least 140 µm, even more preferred 180 µm to 220 µm, formed by an outer epithelial monolayer of trophoblast-like cells surrounding a fluid-filled cavity and at least one inner cluster of cells comprising epiblast-like and hypoblast-like cells. The inventive blastoid may comprise any or all of these properties.

In a further embodiment the blastoid generated by the inventive method can be seeded onto a layer of endometrial cells. The seeding is preferably done in vitro. The blastoid may be allowed to implant into or onto a layer of endometrial cells. This implantation is a process the blastoid can do by itself, if not artificially inhibited. The layer of endometrial cells may be a monolayer.

Preferably the endometrial cells or the endometrium in an in vitro fertilization method have/has been treated with a compound selected from estrogen, estrone, estriol, ethinyl estradiol, 17α-ethylnylestradiol, mestranol, progesterone, a progestin, cAMP, and a Wnt-inhibitor, preferably XAV939, IWP2, PNU-74654, and LF3. Such a treatment improves receptiveness for blastoid or blastocyst implantation into or onto the endometrial cells. In particular preferred is the treatment with a Wnt-inhibitor, preferably XAV939, or any of the Wnt inhibitors mentioned above. This treatment with a Wnt inhibitor can be combined with a treatment with estrogen, estrone, estriol, ethinyl estradiol, 17α-ethylnylestradiol, mestranol, progesterone, a progestin and/or cAMP.

In particular preferred is the inhibition of Wnt for preparing a layer of endometrial cells or an endometrium in the uterus in the course of in vitro fertilization for blastoid or blastocyst implantation. It increases receptiveness of the endometrial cells or endometrium for implantation.

The seeding of the blastoid onto a layer of endometrial cells can be used to study effects on implantation quality, effectiveness and/or inhibition. In general, any stage of the inventive method can be used to study the development or abilities or properties of a blastoid as a model of blastocyst development or abilities or properties.

The inventive method can be used for testing or screening a candidate compound and/or candidate genetic alteration and/or even environmental effects, such as temperature, on having an effect at blastoid formation and/or implantation of a blastoid into a layer of endometrial cell. Such a method may comprise treating the aggregate with at least one candidate compound and/or providing the aggregate with at least one candidate genetic alteration and performing the method of the invention. The effects of this method may be compared to the method without the respective at least one candidate compound and/or at least one candidate genetic alteration and/or altered environmental effect as control comparison. Otherwise, the control comparison is performed likewise as is common for controls in order to evaluate the effect of the at least one candidate compound and/or at least one candidate genetic alteration and/or environmental effects only.

For successful implantation and subsequent development to occur, distinct tissues (polar trophoblast and mural trophoblast) form on each side of the trophoblast cyst. These tissues are thought to play different roles (e.g., adhesion, induction of proliferation) during the interaction with the endometrium and uterine tissues. Accordingly, human blastocysts implant via the polar tissue. The inventive method can be used to study the development of these distinct tissues and implantation therewith. Candidate compounds and/or candidate genetic alterations and/or environmental effects can be studied if they influence this tissue formation or implantation thereof.

For successful implantation and subsequent development to occur, the inner cluster of epiblast-like and hypoblast-like cells secretes molecules that are inducing the outer trophoblasts. These molecular inductions are thought to play different roles (e.g. proliferation, differentiation, mechanical action) to endow trophoblasts with the capacity to interact with the endometrium and uterine tissues. Accordingly, human blastocysts implant via the polar tissue. The inventive method can be used to study the role of the molecular inducers in endowing the trophoblasts to interact with the endometrial and uterine tissues. Candidate molecular inducers and/or candidate genetic alterations and/or environmental effects can be studied if they influence these molecular inducers and their effects on trophoblasts or implantation thereof.

The present invention also provides a blastoid obtainable by a method of the invention. The invention provides a blastoid comprising an outer epithelial monolayer of trophoblast-like cells surrounding at least one fluid-filled cavity and at least one inner cluster of cells comprising epiblast-like and hypoblast-like cells, wherein the outer epithelial monolayer comprises polar trophoblasts that express NR2F2. Any of the above-described property, cell type, tissue type may be part of the inventive blastoid, such as a fluid-filled cavity, which may be free of immobilized cells. It may also be free of disseminated cells or may comprise disseminated cells.

The present invention further provides a kit suitable for culturing a blastoid. Any component or combination thereof mentioned above may be in the kit. The kit may preferably comprise a HIPPO pathway inhibitor, a MEK inhibitor and/or a TGF-beta inhibitor. These compounds are preferably combined in a medium for human pluripotent stem cells (hPSCs). The compounds may be for use in any step as mentioned above. The kit preferably further comprises a Wnt inhibitor, e.g. XAV-939. Any of the above-described inhibitors may be used, with the indicated preferred inhibitors also being preferred for the inventive kit. A particularly preferred HIPPO pathway inhibitor is LPA.

The kit may comprise any compound selected from PD0325901 (a MEK inhibitor), Go6983 (a PKC inhibitor), XAV-939 (a Wnt inhibitor), A83-01 (a TGF-beta inhibitor), or combinations thereof, as preferred examples of a MEK inhibitor, a PKC inhibitor, a Wnt inhibitor and a TGF-beta inhibitor, respectively.

The kit may also comprise a ROCK inhibitor.

The kit may also comprise any growth factor selected from LIF, IGF-1, IL-6, IL-11, FGF2, FGF4 or combinations thereof. LIF may be used as described above. IGF-1 and/or IL-6 and/or IL-11 and/or FGF2 and/or FGF4 may be used to improve aggregate cell growth in a medium of the invention during any one of stages (i), (ii), (iii), or combinations thereof.

The kit may also describe a medium as described above. In particular preferred, the kit comprises insulin.

As discussed above, the present invention has shown that the use of a Wnt inhibitor improves implantation of a blastoid onto a layer of endometrial cells. Accordingly, the invention provides an in vitro method of increasing the potential of implanting a blastoid or blastocyst into a layer of endometrial cells, comprising treating the blastoid or blastocyst with a Wnt inhibitor, preferably XAV939, and contacting the blastoid or blastocyst with the layer of endometrial cells. This method can also be used to test at least one candidate compound and/or at least one candidate genetic alteration and/or environmental effects similar as described above to test thereof on implantation or the development of the blastoid after implantation or the endometrial cells after implantation, e.g. in comparison to a control without such as at least one candidate compound and/or at least one candidate genetic alteration and/or environmental effect.

The improvement of using a Wnt inhibitor can also be used in vivo and/or in vitro during an in vitro fertilization (IVF) method.

Provided is a Wnt inhibitor for use in a method of increasing the chance of a blastocyst implantation during in vitro fertilization, comprising contacting the blastocyst with an endometrium in the presence of the Wnt inhibitor, preferably XAV939; preferably wherein the endometrium is contacted with the Wnt inhibitor topically, systemically or together with the blastocyst. Related thereto, the invention provides a method of increasing the chance of a blastocyst implantation during in vitro fertilization, comprising contacting the blastocyst with an endometrium in the presence of the Wnt inhibitor. Also provided is the use of a Wnt inhibitor for manufacturing a pharmaceutical composition for mediating blastocyst implantation during in vitro fertilization that comprises contacting the blastocyst with an endometrium in the presence of the Wnt inhibitor.

IVF includes contacting an endometrium with an embryo, the embryo could have been grown to a blastocyst stage in vitro. During growth in vitro or during or shortly after contacting the blastocyst with the endometrium, the blastocyst or the endometrium is provided with the Wnt inhibitor to increase the chance of implantation.

Also, the HIPPO pathway inhibitor as discussed improves IVF, similarity as discussed above with regard to the blastoid. For IVF or in general blastocyst preparation, said blastocyst or any preceding stage, e.g. a 1-cell stage, 2-cells stage, 4-cells stage, 8-cells stage or 16-cells stage, or morula stage, can be treated with the HIPPO pathway inhibitor. The invention provides a HIPPO pathway inhibitor for use in a method of producing a blastocyst suitable for in vitro fertilization, comprising treating an embryo in an early stage, selected from the 1-cell stage, 2-cells stage, 4-cells stage, 8-cells stage or 16-cells stage, or morula stage or blastocyst stage until a mature blastocyst stage, with the HIPPO pathway inhibitor, especially preferred a ligand of the lysophosphatidic acid (LPA) receptor, even more preferred LPA, and letting the embryo in a 1-cell stage, 2-cells stage, 4-cells stage, 8-cells stage or 16-cells stage or morula stage grow into the blastocyst stage or letting the embryo in the blastocyst stage grow into a more mature blastocyst stage. Related thereto, the invention provides a method of producing a blastocyst suitable for in vitro fertilization, comprising treating an embryo in an early stage, selected from the 1-cell stage, 2-cells stage, 4-cells stage, 8-cells stage or 16-cells stage or morula stage or blastocyst stage until a mature blastocyst stage, with the HIPPO pathway inhibitor and letting the embryo in a 1-cell stage, 2-cells stage, 4-cells stage, 8-cells stage or 16-cells stage or morula stage grow into the blastocyst stage or letting the embryo in the blastocyst stage grow into a more mature blastocyst stage. Also provided is the use of a HIPPO pathway inhibitor for the manufacture of a pharmaceutical composition for producing a blastocyst suitable for in vitro fertilization, which comprises treating an embryo in an early stage, selected from the 1-cell stage, 2-cells stage, 4-cells stage, 8-cells stage or 16-cells stage or morula stage or blastocyst stage until a mature blastocyst stage, with the HIPPO pathway inhibitor and letting the embryo in a 1-cell stage, 2-cells stage, 4-cells stage, 8-cells stage or 16-cells stage or morula stage grow into the blastocyst stage or letting the embryo in the blastocyst stage grow into a more mature blastocyst stage. For example, the Hippo inhibitor could be used by a patient to increase the chances of getting pregnant by improving the development of the blastocyst in utero, for example, by taking a HIPPO pathway inhibitor a few days after conception and before implantation (e.g. days 6 to 9).

The expression "more mature blastocyst stage" refers to an improvement in maturation by the HIPPO pathway inhibitor. The improvement is to a blastocyst as control or comparison that is maintained or grown under same conditions with the exception of the lack of the HIPPO pathway inhibitor used according to the invention.

The following numbered embodiments are preferred according to the invention:
1. A method of generating a blastoid or a blastocyst-like cell aggregate comprising culturing an aggregate of human pluripotent stem cells (hPSCs) and trophoblast cells in a medium comprising a HIPPO pathway inhibitor in a 3D culture.
2. The method of 1, wherein the aggregate of hPSCs and trophoblasts is generated by culturing aggregated hPSCs in a medium comprising a MEK inhibitor and a TGF-beta inhibitor, and preferably also a HIPPO pathway inhibitor.
3. The method of 2, wherein the aggregated hPSCs are formed by seeding hPSCs and aggregating the seeded hPSCs by culturing in a growth medium, preferably culturing in a growth medium for 12 to 64 hours, and/or preferably wherein the growth medium comprises a ROCK inhibitor, especially preferred the ROCK inhibitor being Y27632.
4. The method of 3, wherein the seeded hPSCs have been treated before the 3D culture, preferably during a 2D culture, with a MEK inhibitor and/or a PKC inhibitor, preferably further comprising a Wnt inhibitor and/or a STAT agonist, preferably wherein treatment is in a 2D culture.
5. The method of 4, wherein the PKC inhibitor is selected from G66983 and Ro-31-8425.
6. The method of any one of 1 to 5, wherein the HIPPO pathway inhibitor is a ligand of the lysophosphatidic acid (LPA) receptor, preferably LPA and/or NAEPA; and/or the MEK inhibitor is PD0325901; and/or the TGF-beta inhibitor is A83-1.
7. The method of any one of 1 to 6, wherein the medium for culturing in a 3D culture and/or the medium of culturing aggregated hPSCs as set forth in embodiment 2 further comprises a STAT3 agonist, preferably leukemia inhibitory factor (LIF).
8. The method of any one of 1 to 7, wherein the 3D culture is by culturing in a non-adherent vessel, preferably by culturing in microwells, especially preferred by microwells comprising a non-adherent surface made of hydrogel.
9. The method of any one of 3 to 8, wherein culturing of hPSCs in the growth medium of embodiment 3 comprises seeding 1 to 200 hPSCs, preferably 20 to 150 hPSCs, especially preferred 30 to 60 hPSCs, in a vessel and growing said seeded hPSCs in the growth medium.
10. The method of any one of 1 to 9, wherein the hPSCs and trophoblasts are cultured for at least 1 day, preferably at least 2 days.
11. The method of any one of 2 to 10, wherein the aggregated cells as set forth in embodiment 2 are cultured for at least 1 day, preferably at least 2 days.
12. The method of any one of 1 to 11 comprising culturing the cells at least until formation of a trophectoderm-like tissue, an epiblast-like tissue and a hypoblast-like tissue out of the aggregate of hPSCs and trophoblasts, preferably further at least until formation of an embryonic-abembryonic axis.
13. The method of any one of 1 to 12 comprising culturing the cells at least until formation of a three-dimensional cell aggregate with an overall diameter of at least 100 µm, preferably at least 140 µm, even more preferred 180 µm to 220 µm, formed by an outer epithelial monolayer of trophoblast-like cells surrounding a fluid-filled cavity and at least one inner cluster of cells comprising epiblast-like and hypoblast-like cells.
14. The method of any one of 1 to 13 further comprising seeding the blastoid onto endometrial cells and allowing the blastoid to implant into or onto endometrial cells.
15. The method of 14, wherein the endometrial cells have been treated with a compound selected from estrogen, estrone, estriol, ethinyl estradiol, 17α-ethylnylestradiol, mestranol, progesterone, a progestin, cAMP, and a Wnt-inhibitor, preferably XAV939 and/or LF3.
16. The method of any one of 1 to 15 for testing or screening a candidate compound and/or candidate genetic alteration on having an effect at blastoid formation and/or implantation of a blastoid into a layer of endometrial cell comprising treating the aggregate with at least one candidate compound and/or providing the aggregate with at least one candidate genetic alteration and performing the method of any one of embodiments 1 to 15.
17. A kit suitable for culturing a blastoid, comprising a HIPPO pathway inhibitor, a MEK inhibitor, and a TGF-beta inhibitor; preferably combined in a medium for human pluripotent stem cells (hPSCs).
18. A blastoid obtainable by a method of any one of 1 to 16.
19. A blastoid comprising an outer epithelial monolayer of trophoblast-like cells surrounding at least one fluid-filled cavity and at least one inner cluster of cells comprising epiblast-like and hypoblast-like cells, wherein the outer epithelial monolayer comprises polar trophoblasts that express NR2F2.
20. An in vitro method of increasing the potential of implanting a blastoid or blastocyst into a layer of endometrial cells, comprising treating the blastoid or blastocyst with a Wnt inhibitor, preferably XAV939 and/or LF3, and contacting the blastoid or blastocyst with the layer of endometrial cells.
21. A Wnt inhibitor for use in a method of increasing the chance of a blastocyst implantation, e.g. during in vitro fertilization or during natural pregnancy, comprising contacting the blastocyst with an endometrium in the presence of the Wnt inhibitor or stimulating the endometrial cells in the absence of a blastocyst with a Wnt inhibitor, preferably XAV939 and/or LF3; preferably wherein the endometrium is contacted with the Wnt inhibitor topically, systemically or together with the blastocyst.
22. A HIPPO pathway inhibitor for use in a method of producing a blastocyst suitable for in vitro fertilization, comprising treating an embryo in an early stage, selected from the 1-cell stage, 2-cells stage, 4-cells stage, 8-cells stage or 16-cells stage or morula stage or blastocyst stage until a mature blastocyst stage, with the HIPPO pathway inhibitor, especially preferred a ligand of the lysophosphatidic acid (LPA) receptor, even more preferred LPA, and letting the embryo in a 1-cell stage, 2-cells stage, 4-cells stage, 8-cells stage or 16-cells stage or morula stage grow into the blastocyst stage or letting the embryo in the blastocyst stage grow into a more mature blastocyst stage.
23. A method of forming a trophosphere comprising culturing hPSCs in the presence of a MST1/2 inhibitor, preferably XMU-MP-1, and/or a STAT inhibitor, preferably SC144.
24. The method of 23 further performed as set forth in any one of embodiments 1 to 16.
25. A method of contraception, comprising administering to a patient and/or contacting an embryo in vivo with a MST1/2 inhibitor, preferably XMU-MP-1, and/or a STAT inhibitor, preferably SC144.
26. The method of claim 25, wherein the MST1/2 inhibitor, preferably XMU-MP-1, and/or a STAT inhibitor, preferably SC144, is administered to a patient 1 to 9 days after conception, preferably 2 to 7 days after conception, in particular preferred 3 to 6 days after conception.
27. A MST1/2 inhibitor, preferably XMU-MP-1, and/or a STAT inhibitor, preferably SC144, for use in a method of contraception, preferably according to embodiment 25 or 26.28. A MST1/2 inhibitor, preferably XMU-MP-1, and/or a STAT inhibitor, preferably SC144, for use in the manufacture of a contraceptive.

Throughout the present disclosure, the articles "a", "an", and "the" are used herein to refer to one or to more than one (i.e., to at least one) of the grammatical object of the article.

As used herein, words of approximation such as, without limitation, "about", "substantial" or "substantially" refer to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by e.g. ±10%.

As used herein, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The "comprising" expressions when used on an element in combination with a numerical range of a certain value of that element means that the element is limited to that range while "comprising" still relates to the optional presence of other elements. E.g. the element with a range may be subject to an implicit proviso excluding the presence of that element in an amount outside of that range. As used herein, the phrase "consisting essentially of" requires the specified integer(s) or steps as well as those that do not materially affect the character or function of the claimed invention. As used herein, the closed term "consisting" is used to indicate the presence of the recited elements only.

The present invention is further illustrated by the following examples, without being limited to these embodiments of the invention.

### Figures

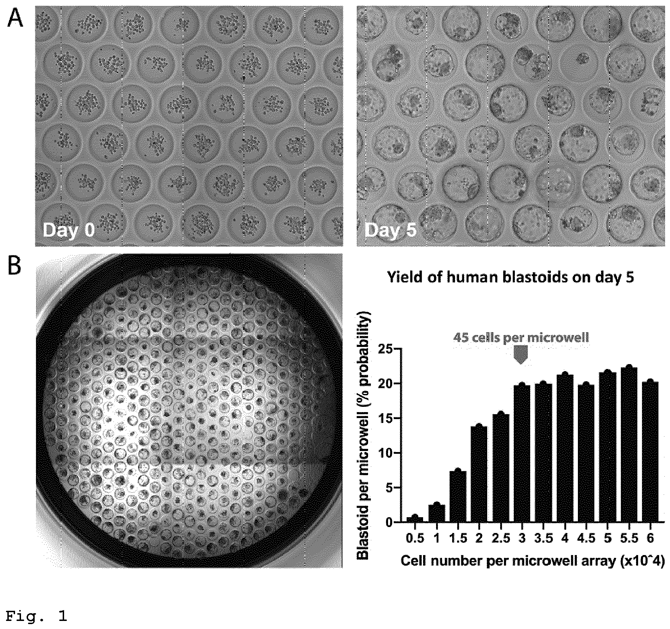

**Figure 1****: Formation of human blastoids. A.** Human pluripotent stem cells (hPSCs) are dissociated into single cells and seeded onto a microwell array. Under specific conditions, within 5 days, hPSCs aggregate and form a blastoid. **B.** The percentage of microwells including a blastoid depends on the initial number of cells that is initially seeded within the microwell.

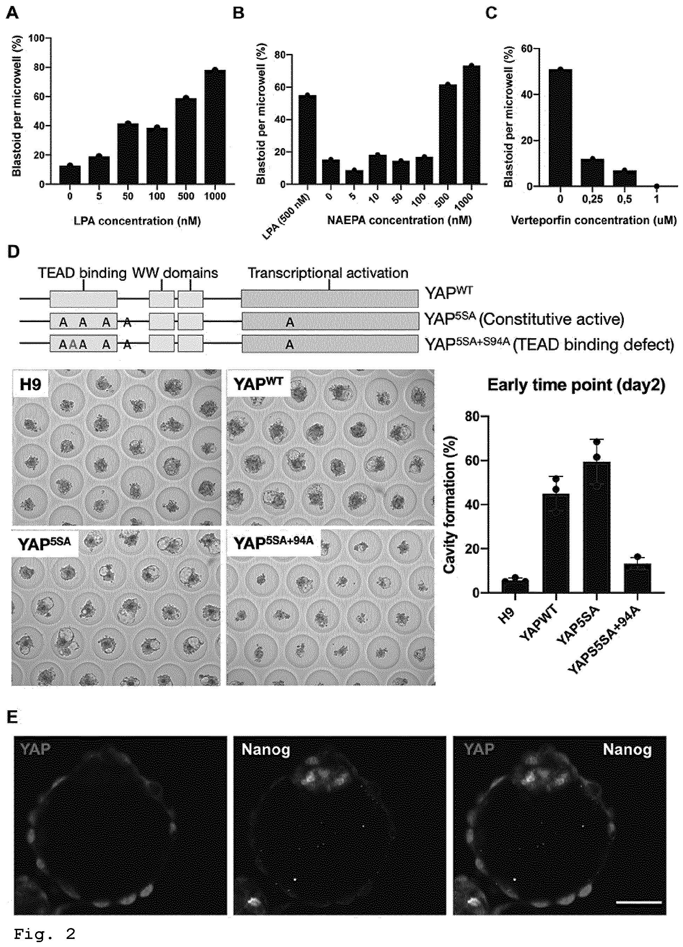

**Figure 2****: Modulation of the Hippo pathway using small molecules and genetic approaches regulates human blastoid formation.** The Hippo pathway can be inhibited using Lysophosphatidic acid (LPA), NAEPA, and the YAP-TEAD complex can be suppressed using Verteporfin, which respectively increases and decreases human blastoid formation. The inhibition of the Hippo pathway can be mimicked using genetic overexpression of YAP-WT, YAP-5SA (constitutive active), which increases the formation of human blastoids. The activation of the hippo pathway can be mimicked by suppressing the formation of the YAP-TEAD complex using YAP-5SA+S94A (TEAD binding defect), which decreases the formation of human blastoids. Immunofluorescence staining of the YAP protein shows a nuclear localization only in the trophectoderm-like cells, not in the epiblast- (Nanog positive cells) and hypoblast-like cells.

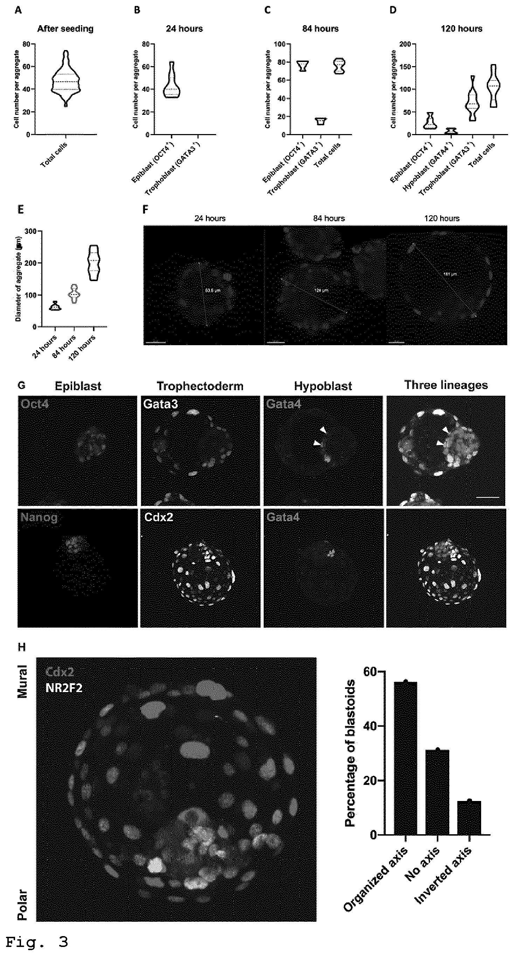

**Figure 3****. Evolution of the number of cells and overall aggregate size during blastoid formation. A.** Upon seeding of the naive hPSCs onto the microwell array, each microwell contains an average of 45 cells. **B.** After 24 hours, the cellular aggregates contain in average 45 cells that are all expressing the epiblast transcription factor Oct4. At this timepoint, cells do not express the trophoblast transcription factor GATA3. **C.** Between 24 and 84 hours, the number of cells increase from an average of 45 to an average of 80, and trophoblast cells expressing GATA3 appear. **D.** Blastoids have fully formed by 120 hours by generating analogs of the three founding cell lineages: OCT4+ epiblast-like cells, GATA4+ hypoblast-like cells, and GATA3+ trophectoderm-like cells. Similar to the human blastocyst, the average total number of cells is 120 and the most abundant lineage is trophoblast cells. **E, F.** Evolution of the overall size of the cellular aggregates during blastoid formation. After 24 hours, the cellular aggregates have an overall diameter of 65 micrometers. This average diameter progressively increases to 200 micrometers after 120 hours (**E**). Pictures of one representative aggregate stained for the nuclear dye Hoechst at 24 hours, 84 hours, and 120 hours (**F**). **G.** Once formed, human blastoids comprise analogs of the three founding lineages: An inner cluster of epiblast-like cells and hypoblast-like cells forms that is characterized by the expression of Oct4 and Nanog, and Gata4, respectively. The outer layer of the human blastoid is formed by a monolayer of trophectoderm-like cells characterized by the expression of Gata3 and Cdx2. **H.** Upon culture of the human blastoids for 5 days, the trophoblasts that are in contact with the inner cluster, termed polar trophoblasts, start expressing NR2F2, while the mural cells do not express it. The polar trophoblasts are known to mediate the initial attachment of the human blastocyst to the uterus. The scale bars are 25 micrometers.
**Figure 4****. Evolution of the structure of the cellular aggregates during blastoid formation. A.** After 24 hours, the cellular aggregates have an overall diameter of 65 micrometers and contain cells that all express the transcription factor Oct4 (see also Figure 3B). The cells in the periphery of the aggregate express higher levels of PKC, a marker of the apical domain. **B.** After 84 hours, the outer cells have reinforced the formation of membrane domains expressing PKC, while the inner cells do not form such domains. The top row shows a cross section of an aggregate. The bottom row shows a full 3D projection of an aggregate. **C, D.** The formation of membrane domains expressing PKC coincides with the appearance of cells expressing the trophoblast transcription factor Cdx2 and with formation of small fluid-filled cavities (**C**) that coalesce to ultimately form a unique cavity at 120 hours (**D**).

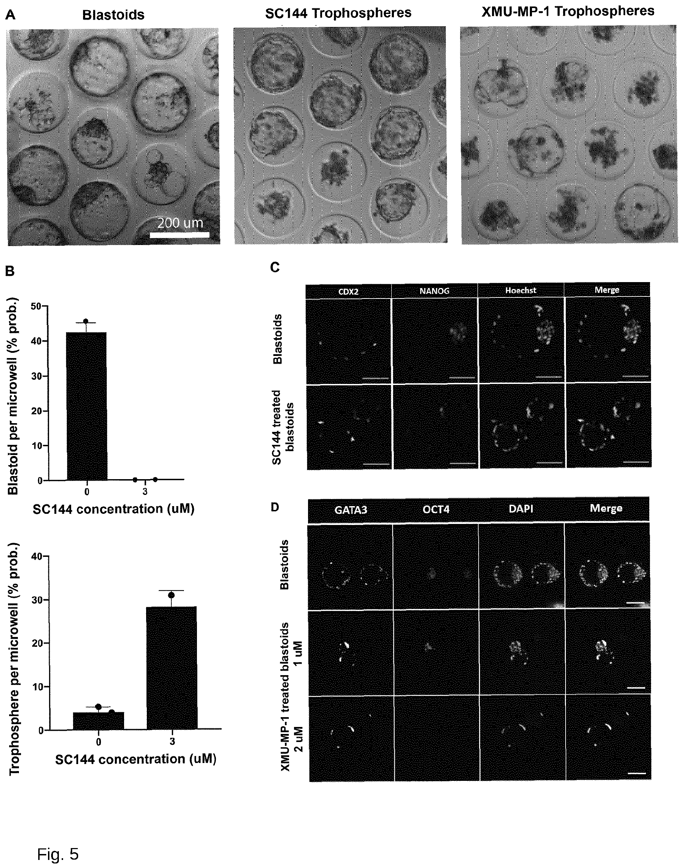

**Figure 5****: Formation of trophospheres by preventing the formation of the inner cluster. A.** The inhibition of the STAT pathway using the small molecule SC144 or of the Hippo pathway using the small molecules XMU-MP-1 results in the formation of blastoids containing less or no inner cell cluster although trophectoderm-like cells are generated that form a cyst with a fluid-filled cavity. This points at the importance of these pathways in balancing or maintaining the number of epiblast-like cells and hypoblast-like cells. **B-D.** The use of 3 uM of the STAT inhibitor SC144 for 4 days results in a decrease in the formation of blastoids and an increase in the formation of trophospheres that contain either no or few epiblast-like cells and hypoblast-like cells. The use of 2 uM of the Hippo inhibitor XMU-MP-1 for 4 days results in the formation of trophospheres that contain either no or few epiblast-like cells and hypoblast-like cells.

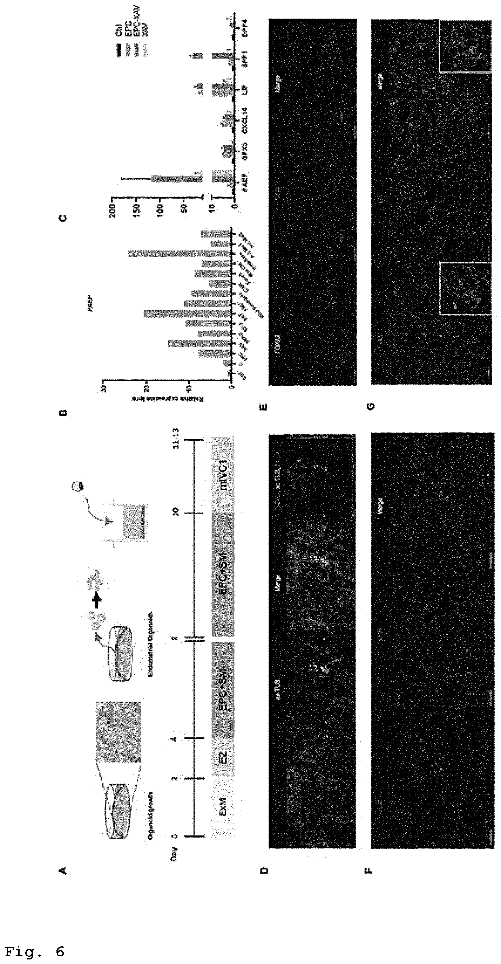

**Figure 6****. Formation of an open-faced endometrial organoid and its stimulation to mimic the Window Of Implantation. A.** Open-faced endometrial organoids are formed by first expanding human endometrial organoids using 3D Matrigel culture, as previously published. These human endometrial organoids are known to recapitulate molecular features of the Window Of Implantation (WOI) upon exposure to a combination of Estrogen, Progesterone and Cyclic adenosine monophosphate (cAMP). This combination is referred to as EPC. In a second step, organoids are dissociated and seeded in 2D to form an open-faced monolayer of endometrial cells. This open-faced monolayer makes the deposition of blastocyst or blastoids easy for assessing the potential to recapitulate aspects of implantation in vitro. **B.** Upon Wnt inhibition with either XAV939 (XAV), or (PKF118-310) PKF, the endometrial cells increase the expression of the gene PAEP, a known marker of the WOI. **C.** The expression of additional markers of the WOI including LIF and SPP1 are also upregulated upon Wnt inhibition and EPC stimulation as compared to EPC stimulation alone. **D.** Immunofluorescence shows that the open-faced endometrial organoids contain subpopulations of ciliated cells (cells positive for acetylated alpha tubulin), (**E**) glandular cells (FOXA2+ cells) and (**F**) proliferating cells (cells positive for EdU incorporation). **G.** The endometrial cells stimulated with EPC and Wnt inhibitors express higher levels of PAEP at the protein level as compared to non-stimulated organoids.

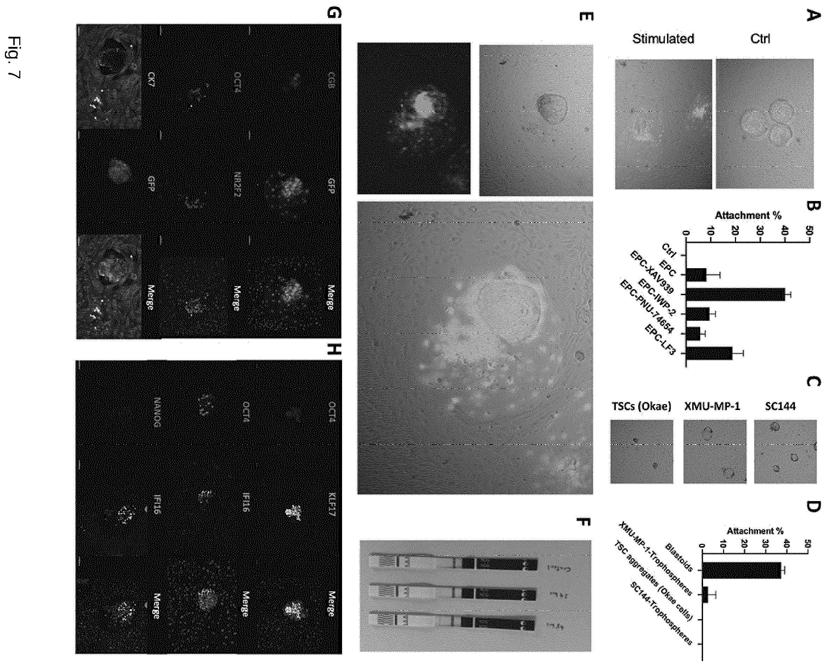

**Figure 7****. The in vitro combination of human blastoids and open-faced endometrial organoid recapitulates features of blastocyst implantation into the uterus.** Human blastoids recapitulate features of implantation into the endometrium. **A, B.** The interaction between human blastoids and open-faced organoids necessitate to stimulate the endometrial cells. Human blastoids fail to attach and invade into unstimulated endometrial cells that do not mimic the Window Of Implantation (WOI) (**top**) but attach and invade endometrial cells that are stimulated (**bottom**). **B.** The combination of EPC stimulation and Wnt inhibition using the inhibitors XAV939, IWP-2, PNU-74654, and LF3 increases the potential of endometrial cells to interact with human blastoids. **C-E.** The interaction between human blastoids and open-faced endometrial organoids necessitates a specific trophoblast state. The presence of the STAT inhibitor SC144 during human blastoid formation induces the formation of trophospheres with limited or no epiblast-like cells and hypoblast-like cells. The presence of the Hippo inhibitor XMU-MP-1 during human blastoid formation induces the formation of trophospheres with limited or no epiblast-like cells and hypoblast-like cells. Human trophoblast stem cells (Okae et al, 2018, doi: 10.1016/j.stem.2017.11.004) can form cytotrophoblast aggregates reflecting the post-implantation stage. SC144-trophospheres, XMU-MP-1-trophospheres and cytotrophoblast aggregates fail to attach and invade stimulated endometrial cells (**C, D**). On the contrary, human blastoids are capable of attaching and invading stimulated endometrial cells and attach through the polar region (**D, E**). **F.** Upon attachment and invasion, human chorionic gonadotrophin (hCGB), which is the hormone used to assess a clinical pregnancy, is detected into the culture medium after 24 and 48 hours (middle and right pregnancy test strips, respectively). Levels are undetectable if human blastoids do not attach and invade unstimulated endometrial cells (left test strip). **G.** Immunostaining shows that the hormone hCGB is only produced by some cells of the attached blastoids. Immunostaining also shows that the attached blastoids form numerous cells that are positive for NR2F2, a marker for polar and post-implantation trophoblasts. These trophoblasts are different from the Oct4-positive cells characteristic of the epiblast. Attached human blastoids contain trophoblasts expressing CK7, a marker of post-implantation trophoblasts. **H.** Upon attachment and invasion into the endometrial cells, blastoids form cells positive for the epiblast markers Oct4, Klf17, Nanog, and IFI16.

### Examples

### Example 1: Formation of an aggregate of hPSCs.

The formation of blastoids relies on the aggregation of an optimal number of hPSCs. This can be achieved by seeding specific numbers of hPSCs onto a microwell array made of non-adherent hydrogel and containing numerous microwells of, for example, 200 micrometers (**figure 1A**). We observed that human blastoids preferentially form from aggregates of 20-100 cells, which favors the formation of aggregates capable of forming a cavity (**figure 1B**). The phenomena is quantified by measuring the number of structures that form a cavity, and of blastoids defined as a three-dimensional structure with an overall diameter comprised between 180 and 220 micrometers, formed by an epithelial monolayer of trophoblast-like cells surrounding a unique fluid-filled cavity and (an) inner cluster(s) of cells composed of epiblast- and hypoblast-like cells. The timing of molecular stimulation of the aggregate also determines the level of cellular specification and self-organization within this aggregate. We observed that the initial aggregation of the cells in B27N2 medium, followed, 24-48 hours later, by the replacement of the culture medium by a B27N2 medium containing A83-1, PD0325901, LIF is sufficient for the aggregate to form the three cell types and the outer trophoblast cyst that contains a single inner cell cluster of epiblast and hypoblast cells. Under these conditions, human blastoids form within 5 days (**figure 1B**).

### Example 2: Inhibition of the HIPPO pathway by the use of either small molecules or genetic approaches.

The activity of the HIPPO pathway is essential for blastoid formation. The stimulation of hPSCs aggregates using the small molecule LPA or NAEPA, inhibitors of the HIPPO pathway activity, leads to the transition from a solid aggregate of cells to a cystic structure including a single fluid-filled cavity. This fluid-filled cavity is lined by cells expressing trophectoderm markers, including CDX2 and GATA3, and comprises a single cluster of cells expressing markers of the epiblast, including NANOG and OCT4, and cells expressing markers of the hypoblast, including GATA4. The necessity of modulating the activity of the HIPPO pathway to form blastoids is also assessed by the effect observed upon treatment of the aggregate of hPSCs with the YAP-specific inhibitor, Verteporfin. This small molecule totally prevents the formation of a cavity (1 µM) (**Figure 2C**). In accordance with the role of the HIPPO pathway in human blastoid formation, the HIPPO pathway effector YAP is translocated into the nuclei of the outer trophectoderm-like cells, while it is not located in the nuclei of the inner cell cluster (**Figure 2E**). Finally, overexpression of wild-type and constitutive active YAP (YAP 5SA) facilitates the cavity formation in the early stage of blastoid formation (Day 2) (**Figure 2D**). On the contrary, overexpression of YAP with the mutation in the TEAD binding site (YAP S94A), which is known to prevent the activation of the HIPPO target genes, does not show any positive effect on cavity formation. Thus, consistent with the data generated using small molecules, the modulation of the HIPPO pathway is necessary for the genetic activation of the trophectoderm program and the formation of a blastocoel-like cavity.

### Example 3: Concomitant specification and morphogenesis of trophectoderm-like, epiblast-like, and hypoblast-like tissues.

Inhibition of LIF-STAT pathway by treatment of chemical inhibitor SC-144 induced the formation of mono-layer cavitated aggregate that does not form an inner cluster of Epi-/Hypo-like cells. The maturation of lineage specification of outer trophectoderm cells were also compromised by the SC-144 treatment, which indicates the crucial role of cell-cell communication among three lineages for blastoid formation.

### Example 4: Spontaneous formation of the embryonic-abembryonic axis.

In the late human blastocyst, the polar trophectoderm that juxtapose the epiblast starts expressing the transcription factor NR2F2, thus marking the region that will mediate the implantation into the uterus. Similarly, upon in vitro culture the trophectoderm-like tissues of blastoids spontaneously form a distinct region characterized by the expression of NR2F2. On the contrary, the mural trophectoderm opposite the fluid-filled cavity does not express NR2F2 (**Figure 3H**). Thus, similar to blastocyst, blastoids are capable of spontaneously forming the region that mediate the implantation, and of forming an axis.

### Example 5: Differentiation of endometrial cells into receptive cells mimicking the window of implantation.

In order to model blastocyst implantation in vitro, we have defined conditions under which endometrial cells originating from organoids can be deposited in 2D culture plates and stimulated with molecules that allow them differentiate into the cells lining the uterus at the time of implantation (so called Window of Implantation, WOI). We discovered that, similar to hormones (Estradiol, Progesterone), the inhibition of the Wnt signaling pathway using multiple small molecules induces an up-regulation of the expression of genes highly expressed during the WOI (**Figure 6**). The inhibition of Wnt increases the expression of PAEP, SPP1, LIF and DPP4, as seen using RTqPCR of endometrial cells cultured with XAV939. The stimulated endometrial cells express PAEP also at the protein level, as shown using immunofluorescence and the cells are proliferating, as seen by the incorporation of EdU. The endometrial cells also contain subpopulations of ciliated cells that specifically express acetylated alpha tubulin and glandular cells that specifically express FOXA2, as seen using immunofluorescence.

### Example 6: Implantation of human blastoids into receptive endometrium organoids.

Upon deposition of human blastoids onto EPC/XAV939-treated endometrial cells, blastoids attach and their cells invade the endometrial layer (**Figure 7**). Upon attachment and invasion, the blastoid cells maintain the expression of the pluripotency transcription factor Oct4. When endometrial cells are not stimulated with EPC/XAV939, blastoids largely fail to attach and to invade the endometrial layer. When blastoids largely fail to form an inner cluster of epiblast-like cells and hypoblast-like cells (SC144-Trophospheres, XMU-MP-1-Trophospheres), they largely fail to attach and to invade the endometrial layer. Aggregates formed from human trophoblast stem cells (Okae et al. 2018) that reflect post-implantation cytotrophoblasts fail to attach and invade the endometrial layer.

## Claims

1. A method of generating a blastoid or a blastocyst-like cell aggregate comprising culturing an aggregate of human pluripotent stem cells (hPSCs) and trophoblast cells in a medium comprising a HIPPO pathway inhibitor in a 3D culture.

2. The method of claim 1, wherein the aggregate of hPSCs and trophoblasts is generated by culturing aggregated hPSCs in a medium comprising a a MEK inhibitor and a TGF-beta inhibitor and preferably also a HIPPO pathway inhibitor.

3. The method of claim 2, wherein the aggregated hPSCs are formed by seeding hPSCs and aggregating the seeded hPSCs by culturing in a growth medium, preferably culturing in a growth medium for 12 to 64 hours, and/or preferably wherein the growth medium comprises a ROCK inhibitor, especially preferred the ROCK inhibitor being Y27632.

4. The method of claim 3, wherein the seeded hPSCs have been treated with a MEK inhibitor and/or a PKC inhibitor, preferably further comprising a Wnt inhibitor and/or a STAT agonist, preferably wherein treatment is in a 2D culture.

5. The method of claim 4, wherein the PKC inhibitor is selected from Gö6983 and Ro-31-8425.

6. The method of any one of claims 1 to 5, wherein the HIPPO pathway inhibitor is a ligand of the lysophosphatidic acid (LPA) receptor, preferably LPA or NAEPA; and/or the MEK inhibitor is PD0325901; and/or the TGF-beta inhibitor is A83-1.

7. The method of any one of claims 1 to 6 comprising culturing the cells at least until formation of a trophectoderm-like tissue, an epiblast-like tissue and a hypoblast-like tissue out of the aggregate of hPSCs and trophoblasts, preferably further at least until formation of an embryonic-abembryonic axis; and/or culturing the cells at least until formation of a three-dimensional cell aggregate with an overall diameter of at least 100 µm, preferably at least 140 µm, even more preferred 180 µm to 220 µm, formed by an outer epithelial monolayer of trophoblast-like cells surrounding a fluid-filled cavity and at least one inner cluster of cells comprising epiblast-like and hypoblast-like cells.

8. The method of any one of claims 1 to 7 further comprising the stimulation of endometrial cells with a compound selected from estrogen, estrone, estriol, ethinyl estradiol, 17α-ethylnylestradiol, mestranol, progesterone, a progestin, cAMP, and a Wnt-inhibitor, preferably XAV939 or LF3 and the seeding of the seeding of the blastoid onto the layer of stimulated endometrial cells that allows the blastoid to attach and invade the layer of endometrial cells.

9. The method of any one of claims 1 to 8 for testing or screening a candidate compound and/or candidate genetic alteration on having an effect at blastoid formation and/or implantation of a blastoid into a layer of endometrial cell comprising treating the aggregate with at least one candidate compound and/or providing the aggregate with at least one candidate genetic alteration and performing the method of any one of claims 1 to 8.

10. A kit suitable for culturing a blastoid, comprising a HIPPO pathway inhibitor, a MEK inhibitor, and a TGF-beta inhibitor; preferably combined in a medium for human pluripotent stem cells (hPSCs).

11. A blastoid obtainable by a method of any one of claims 1 to 10.

12. A blastoid comprising an outer epithelial monolayer of trophoblast-like cells surrounding at least one fluid-filled cavity and at least one inner cluster of cells comprising epiblast-like and hypoblast-like cells, wherein the outer epithelial monolayer comprises polar trophoblasts that express NR2F2.

13. An in vitro method of increasing the potential of implanting a blastoid or blastocyst into a layer of endometrial cells, comprising treating the blastoid or blastocyst with a Wnt inhibitor, preferably XAV939 and/or LF3, and contacting the blastoid or blastocyst with the layer of endometrial cells.

14. A Wnt inhibitor for use in a method of increasing the chance of a blastocyst implantation, comprising contacting the blastocyst with an endometrium in the presence of the Wnt inhibitor, preferably XAV939 and/or LF3, or comprising stimulating the endometrium with a Wnt inhibitor before transferring the blastocyst to the endometrium; preferably wherein the endometrium is contacted with the Wnt inhibitor topically, systemically or together with the blastocyst.

15. A HIPPO pathway inhibitor for use in a method of producing a blastocyst suitable for in vitro fertilization, comprising treating an embryo in an early stage, selected from the 1-cell stage, 2-cells stage, 4-cells stage, 8-cells stage or 16-cells stage or morula stage or blastocyst stage until a mature blastocyst stage, with the HIPPO pathway inhibitor, especially preferred a ligand of the lysophosphatidic acid (LPA) receptor, even more preferred LPA, and letting the embryo in a 1-cell stage, 2-cells stage, 4-cells stage, 8-cells stage or 16-cells stage or morula stage grow into the blastocyst stage or letting the embryo in the blastocyst stage grow into a more mature blastocyst stage.
